(19) Europäisches Patentamt
European Patent Office
Office européen des brevets

(11) Numéro de publication: **0 057 491**
**B1**

(12) # FASCICULE DE BREVET EUROPEEN

(45) Date de publication du fascicule du brevet: **01.08.84**

(51) Int. Cl.[3]: **C 07 D 307/32**, C 07 D 307/93 // C07C59/74, C07F9/54

(21) Numéro de dépôt: **82200366.1**

(22) Date de dépôt: **03.06.80**

(60) Numéro de publication de la demande initiale en application de l'article 76 CBE:

(54) **Nouveaux dérivés halogénés du 5-hydroxy tétrahydrofuran 2-one.**

(30) Priorité: **06.06.79 FR 7914425**

(43) Date de publication de la demande: **11.08.82 Bulletin 82/32**

(45) Mention de la délivrance du brevet: **01.08.84 Bulletin 84/31**

(84) Etats contractants désignés: **AT BE CH DE GB IT LI LU NL SE**

(56) *Documents cités:*
**EP - A - 0 037 327**

**JOURNAL OF ORGANIC CHEMISTRY, vol. 39, no. 1, 11 janvier 1974 WASHINGTON (US) K. OHGA et al.: "Photoinduced Addition of Isopropyl Alcohol to alpha, beta-Unsaturated Lactones" pages 106-108**

(73) Titulaire: **ROUSSEL-UCLAF, 35, boulevard des Invalides, F-75007 Paris (FR)**

(72) Inventeur: **Martel, Jacques, 15, rue Douvillez, F-93140 Bondy (FR)**
Inventeur: **Tessier, Jean, 30, rue Jean Moulin, F-94300 Vincennes (FR)**
Inventeur: **Demoute, Jean-Pierre, 249 bis, rue de Rosny, F-93100 Montreuil-sous-Bois (FR)**

(74) Mandataire: **Vieillefosse, Jean-Claude et al, ROUSSEL-UCLAF Boîte postale no. 9 111, route de Noisy, F-93230 Romainville (FR)**

EP 0 057 491 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un acide penténoïque substitué, son procédé de préparation et son application à la préparation de dérivés cyclopropaniques substitués.

L'invention a tout d'abord pour objet les composés de formule générale (VIII):

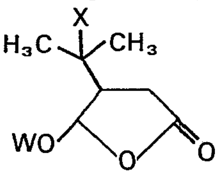

(VIII)

dans laquelle X représente un atome d'halogène et notamment un atome de brome ou de chlore et W représente de l'hydrogène ou un radical $R_1$, $R_1$ représentant le reste d'un alcool $R_1OH$, éventuellement chiral.

Parmi les valeurs préférées de X, on peut citer les atomes de brome et de chlore.

L'invention a également pour objet un procédé de préparation des composés de formule (VIII) tels que définis à la revendication 1, caractérisé en ce que l'on fait réagir en milieu anhydre l'acide 3-formyl-4-méthylpent-3-én-1-oïque, en milieu anhydre avec un hydracide HX, dans lequel X représente un atome d'halogène, en présence d'un hydrogénure du lithium LiX, au sein d'un solvant organique pour obtenir un composé de formule (VIII'):

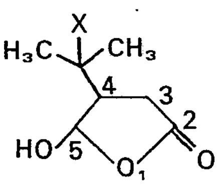

(VIII')

que l'on fait réagir soit avec un alcool $R_1OH$ achiral pour obtenir le composé (VIII") racémique:

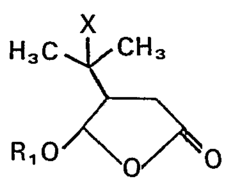

(VIII")

qui, comme le dérivé (VIII'), présente une relation trans entre les substituants en 4 et en 5 de la tétrahydrofurannone, soit avec un alcool $R_1OH$ chiral, le composé (VIII") étant alors obtenu sous la forme d'un mélange des deux diastéréo-isomères prévisibles, mélange que l'on peut séparer en ses constituants par des procédés physiques.

L'invention a plus particulièrement pour objet un procédé de préparation des composés de formule (VIII), caractérisé en ce que l'on fait réagir sur l'acide 3-formyl-4-méthylpent-3-én-1-oïque des couples BrH/BrLi ou ClH/ClLi au sein de l'éther éthylique anhydre, ainsi qu'un procédé, caractérisé en ce que l'on fait réagir l'alcool $R_1OH$ achiral ou chiral et le composé (VIII') en présence d'acide paratoluènesulfonique.

Selon le procédé de l'invention, les deux diastéréo-isomères provenant de l'action de l'alcool chiral $R_1OH$ sur le racémate de composé (VI) sont séparés par chromatographie ou bien par cristallisation.

L'acide 3-formyl-4-méthylpent-3-én-1-oïque utilisé comme produit de départ du procédé de l'invention est un produit décrit et revendiqué dans la demande de brevet européen N° 0023849.

Ce produit peut être préparé selon un procédé, caractérisé en ce que l'on fait réagir un dérivé de formule (II):

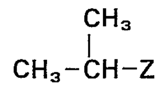

(II)

dans laquelle Z représente un groupement électro-attracteur, avec un dérivé de formule (III):

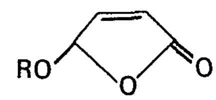

(III)

dans laquelle R représente le reste R d'un alcool ROH, puis soumet le composé résultant de formule (IV):

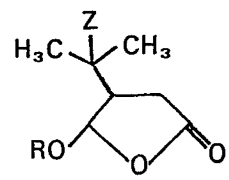

(IV)

à l'action d'un agent basique.

Pour préparer l'acide 3-formyl-4-méthylpent-3-én-1-oïque, on peut utiliser un procédé tel que décrit précédemment, caractérisé en ce que le groupement Z est un groupement $NO_2$ et en ce que l'on fait réagir les composés (II) et (III) en présence d'une base choisie dans le groupe constitué par les amines secondaires, les amines tertiaires, les hydroxydes d'ammonium quaternaires et les carbonates alcalins.

Comme amine tertiaire en présence de laquelle on fait réagir les composés (II) et (III), on utilise avantageusement la triéthylamine.

Pour préparer l'acide 3-formyl-4-méthylpent-3-én-1-oïque, on peut utiliser un procédé tel que décrit précédemment, caractérisé en ce que le groupement Z est un groupement hydroxyle et en ce que l'on fait réagir les composés (II) et (III) sous irradiation ou en présence d'un promoteur radicalaire.

Comme promoteur radicalaire, on utilise avantageusement le peroxyde de benzoyle, l'azobisisobutyronitrile, le peroxyde de diterbutyle, le perbenzoate de terbutyle, le peroxyde de dilauryle.

Pour préparer l'acide 3-formyl-4-méthylpent-3-én-1-oïque, on peut utiliser un procédé tel que décrit précédemment, caractérisé en ce que le groupement Z est un groupement arylsulfonyle et en ce que l'on fait réagir les composés (II) et (III) en présence d'une base forte au sein d'un solvant organique.

Le radical aryle du groupement arylsulfonyle est notamment un radical paratolyle.

Pour préparer l'acide 3-formyl-4-méthylpent-3-én-1-oïque, on peut utiliser un procédé tel que décrit précédemment, caractérisé en ce que le groupement Z est un groupement halogénotriarylphosphonio et en ce que l'on fait réagir les composés (II) et (III) en présence d'une base forte au sein d'un solvant organique.

Le groupement halogénotriarylphosphonio est notamment le radical iodotriphénylphosphonio.

Pour préparer l'acide 3-formyl-4-méthylpent-3-én-1-oïque, on peut utiliser un procédé de préparation tel que défini précédemment, dans lequel le groupement Z est un groupement arylsulfonyle ou un groupement halogénotriarylphosphonio, caractérisé en ce que la base forte est choisie dans le groupe constitué par les alkyllithiens, les amidures alcalins, les hydrures alcalins, les alcoolates alcalins, et en ce que le solvant utilisé est choisi dans le groupe constitué par le tétrahydrofuranne, le diméthylsulfoxyde, le diméthoxyéthane, le diméthylformamide, l'hexaméthylphosphoramide, les hydrocarbures aromatiques et des cycloalcanes.

Lorsque Z est un groupement arylsulfonyle ou un groupement halogénotriarylphosphonio, la réaction entre les composés (II) et (III) est effectuée avantageusement en présence de butyllithium au sein d'un mélange de tétrahydrofuranne et de cyclohexane.

L'agent basique que l'on fait réagir sur les composés (IV) est notamment le carbonate de sodium utilisé en milieu hydrométhanolique.

L'invention a en outre pour objet l'application des composés de formule générale (VIII') à la préparation des composés de formule (V'A):

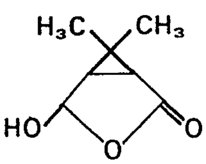

(V'A)

caractérisé en ce que l'on soumet un composé de formule (VIII') à l'action d'un alcool $R_1OH$ pour obtenir un composé de formule (VIII") que l'on soumet à l'action d'un agent basique pour obtenir un composé bicyclique de formule (V'):

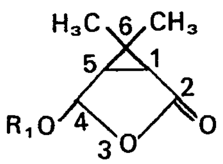

(V')

sous forme racémique ou sous l'une ou l'autre de ses formes énantiomériques selon que $R_1$ est respectivement un reste achiral ou chiral, la configuration absolue en position 4 de ce composé ainsi que celles qui en découlent en positions 1 et 5 étant alors déterminées par la stéréochimie du diastéréo-isomère (VIII") mis en œuvre, puis hydrolyse l'éther de formule (V') obtenu en milieu acide, avec rétention totale des configurations absolues, pour obtenir le composé de formule (V'A) correspondant:

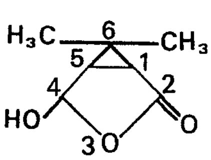

(V'A)

Selon un mode d'application avantageux de l'invention, la transformation du composé (VII) en composé cyclopropanique (V) est effectué à l'aide du butyllithium.

Selon l'application de l'invention, on effectue aussi avantageusement la transformation des tétrahydrofurannones (VII) en composé cyclopropanique (V) soit par la méthode de catalyse en transfert de phase, à l'aide d'un hydroxyde alcalin, d'eau, d'un solvant non miscible à l'eau et d'un ammonium quaternaire, soit à l'aide d'hydrure de sodium.

Dans la méthode de la catalyse par transfert de phase, on utilise avantageusement l'hydroxyde de sodium.

L'hydrolyse des composés (V) en composés (VA) est effectuée en présence d'acide chlorhydrique, avantageusement en milieu hydroacétonique.

Il existait déjà des procédés d'hémisynthèse permettant de préparer la 6,6-diméthyl 4-hydroxy 3-oxabicyclo [3.1.0] hexan-2-one, au départ d'un produit très élaboré: l'acide chrysanthémique (voir par exemple le brevet français N° 1580474).

La société titulaire a maintenant mis au point, par l'intermédiaire du composé (I), un procédé de synthèse totale de la 6,6-diméthyl 4-hydroxy 3-oxabicyclo [3.1.0] hexan-2-one ou de ses éthers, sous forme racémique ou optiquement active. Ce procédé utilise des réactifs facilement accessibles. Ses étapes sont en nombre limité.

L'accès par synthèse totale à un composé tel que la 6,6-diméthyl 4-hydroxy 3-oxabicyclo [3.1.0] hexan-2-one qui sert d'intermédiaire dans la synthèse de nombreux esters insecticides d'activité élevée (voir brevet français N° 2185612) présente un intérêt particulièrement remarquable.

Les exemples suivants illustrent l'invention sans la limiter.

*Exemple 1: 4-(2-Chloroprop-2-yl)-5-hydroxytétrahydrofuran-2-one-dl-trans*

Sous un courant d'acide chlorhydrique anhydre, on agite 1 g d'acide 3-formyl-4-méthylpent-3-én-1-oïque obtenu à l'exemple 1, 2, 3 ou 4, 25 $cm^3$ d'éther éthylique anhydre et 1 g de chlorure de lithium sec pendant 2 h à −30° C, puis 2 h à 0° C, arrête le courant d'acide chlorhydrique et continue l'agitation 48 h à température ambiante, après 54 h de contact, verse le mélange réactionnel dans de l'eau glacée, décante, extrait au benzène, sèche, concentre à sec sous pression réduite, obtient 1,1 g de produit brut, le chromatographie sur silice en éluant par un mélange benzène/acétate d'éthyle 1/1, récupère 545 mg d'un produit qui cristallise, F=80° C.

*Analyse* pour $C_7H_{11}ClO_3$=178,617

| | | | |
|---|---|---|---|
| Calculé: | C 47,07 | H 6,21 | Cl 19,85% |
| Trouvé: | C 47,2 | H 6,2 | Cl 19,5 % |

*Spectre de RMN*

pics à 1,55 et 1,66 ppm attribués aux hydrogènes des méthyles,

pics à 2,42-2,92 ppm attribués aux hydrogènes en positions 3 et 4 du cycle,

pics à 5,82-5,89 ppm attribués à l'hydrogène en 5 du cycle,
pic à 3,83 ppm attribué à l'hydrogène de l'hydroxyle.

*Exemple 2: 4-(2-Bromoprop-2-yl)-5-hydroxytétrahydrofuran-2-one-dl-trans*

On refroidit à −35°C un mélange de 2,35 g d'acide 3-formyl-4-méthylpent-3-én-1-oïque obtenu à l'exemple 1 ou 2, 2,7 g de bromure de lithium sec et 60 cm³ d'éther éthylique anhydre, agite 90 min sous courant d'acide bromhydrique sec, chasse celui-ci par un fort courant d'azote sec tout en maintenant la température entre −30 et −40°C, verse le mélange réactionnel dans de l'eau glacée, extrait au benzène, sèche la phase organique, concentre à sec sous pression réduite sans chauffer, récupère 3,27 g d'une huile qui cristallise, recristallise le produit dans l'éther de pétrole et isole 2,5 g de cristaux blancs fondant à ~75°C.

*Analyse* pour $C_7H_{11}BrO_3$=223,073:

| | | | |
|---|---|---|---|
| Calculé: | C 37,69 | H 4,97 | Br 35,82% |
| Trouvé: | C 37,80 | H 5,20 | Br 35,20% |

*Spectre de RMN*

pics à 1,74 et 1,86 ppm attribués aux hydrogènes des méthyles,
pics à 2,25-3,0 ppm attribués aux hydrogènes en positions 3 et 4 du cycle,
pics à 5,87 et 5,93 ppm attribués à l'hydrogène en position 5 du cycle,
pic à 3,92 ppm attribué à l'hydrogène de l'hydroxyle.

*Exemple 3: 4-(2-Chloroprop-2-yl)-5-[(3-phénoxyphényl)méthoxy]tétrahydrofuran-2-one*

On agite pendant 19 h à température ambiante 393 mg du produit obtenu à l'exemple 1,668 mg d'alcool métaphénoxybenzylique, 20 mg d'acide paratoluènesulfonique et 5 cm³ de benzène, neutralise avec un peu de bicarbonate de sodium, sèche, concentre à sec sous pression réduite, obtient 1,18 g de produit brut, le chromatographie sur silice en éluant par du benzène, récupère 467 mg de produit, qui, recristallisé dans l'éther de pétrole, fond à près de 50°C.

*Analyse* pour $C_{20}H_{21}ClO_4$=360,84

| | | | |
|---|---|---|---|
| Calculé: | C 66,57 | H 5,87 | Cl 9,83% |
| Trouvé: | C 66,60 | H 5,80 | Cl 9,90% |

*Spectre de RMN*

pics à 1,5 et 1,55 ppm attribués aux hydrogènes des méthyles,
pics à 2,55-2,72 ppm attribués aux hydrogènes en positions 3 et 4 du cyclopentyle,
pics à 5,52-5,56 ppm attribués à l'hydrogène en position 5 du cyclopentyle,
pics à 6,92-7,5 ppm attribués aux hydrogènes des noyaux aromatiques,
pics à 4,47-4,66 et 4,77-4,97 ppm attribués aux hydrogènes du méthylène benzylique.

*Exemple 4: 4-(2-Bromoprop-2-yl)-5-[(3-phénoxyphényl)méthoxy]tétrahydrofuran-2-one*

On agite 24 h à température ambiante 5,5 g du produit obtenu à l'exemple 2, 5,2 g d'alcool métaphénoxybenzylique, 50 cm³ de benzène et 270 mg d'acide paratoluènesulfonique, lave le milieu réactionnel avec une solution aqueuse de bicarbonate de sodium, extrait au benzène, sèche, concentre à sec sous pression réduite, obtient environ 10 g d'une huile qui cristallise, recristallise ce produit dans un mélange éther isopropylique/éther de pétrole (7 cm³/5 cm³), isole 5,83 g fondant à 60°C. Une chromatographie des liqueurs mères permet de récupérer 1 g de produit de même pureté.

*Analyse* pour $C_{20}H_{21}BrO_4$:

| | | | |
|---|---|---|---|
| Calculé: | C 59,27 | H 5,22 | Br 19,72% |
| Trouvé: | C 60,10 | H 5,60 | Br 21,50% |

*Spectre de RMN*

pics à 1,67-1,7 ppm attribués aux hydrogènes des méthyles,
pics à 2,22-2,92 ppm attribués aux hydrogènes en positions 3 et 4 du cyclopentyle,
pics à 5,53-5,58 ppm attribués à l'hydrogène en position 5 du cyclopentyle,
pics à 4,48-4,68 et 4,78-4,98 ppm attribués aux hydrogènes du méthylène benzylique,
pics à 6,92-7,58 ppm attribués aux hydrogènes des noyaux aromatiques.

*Exemple 5: 4-(2-Bromoprop-2-yl)-5-[(3-phénoxyphényl)méthylméthoxy]tétrahydrofuran-2-one* (mélange de diastéréo-isomères A et B de structure trans et diastéréo-isomères A et B séparés)

On agite pendant 24 h, à 20/25°C, 6,07 g de bromolactone obtenue à l'exemple 2, 5, 83 g d'alcool (S)-α-méthyl-3-phénoxybenzylique, 300 mg d'acide paratoluènesulfonique et 60 cm³ de benzène et 10 g de déshydratant Actigel coloré, lave le mélange réactionnel avec une solution aqueuse de bicarbonate de sodium, extrait au benzène, sèche, évapore à sec sous pression réduite, obtient 9,5 g de produit brut, chromatographie le produit brut sur silice en éluant au chlorure de méthylène. Des 5 g du mélange pur des diastéréo-isomères ainsi obtenu, on isole 960 mg d'isomère B par cristallisation dans l'éther isopropylique à 30% d'éther de pétrole F≃76°C $(\alpha)_D$=+188°5, (1% benzène); la chromatographie sur silice des liqueurs mères, avec élution au chlorure de méthylène, a permis d'obtenir l'isomère A pur sous la forme d'une huile.

*Exemple 6: 4-(2-Bromoprop-2-yl)-5-isopropyloxytétrahydrofuran-2-one-dl-trans*

Pendant 60 h à température ambiante, on agite 1,95 g du dérivé bromé obtenu à l'exemple 2, 2 cm³ d'isopropanol, 100 mg d'acide paratoluènesulfonique et 30 cm³ de benzène, lave le milieu réactionnel par une solution aqueuse de bicarbonate de sodium, extrait au benzène, sèche, concentre à sec sous pression réduite, obtient 2 g de produit huileux utilisé tel quel au stade suivant.

*Préparation 1: Acide 3-formyl-4-méthylpent-3-én-1-oïque*

*Stade A: 4-(2-Nitroprop-2-yl)-5-méthoxytétrahydrofuran-2-one-trans-dl*

On agite pendant 70 h, à 20/25°C, 9 $cm^3$ de 2-nitropropane, 7 g de 5-méthoxy-2,5-dihydrofuran-2-one-3-ène et 1 $cm^3$ de triéthylamine, lave le mélange réactionnel avec une solution aqueuse de phosphate monosodique, extrait au benzène, sèche, concentre à sec sous pression réduite, reprend le résidu par de l'éther isopropylique, amorce la cristallisation et refroidit à 0°C sous agitation, essore et obtient 8,42 g de produit cristallisé, F≃33°C.

*Analyse* pour $C_8H_{13}NO_5$=203,18

| | | | |
|---|---|---|---|
| Calculé: | C 47,29 | H 6,45 | N 6,89% |
| Trouvé: | C 47,10 | H 6,50 | N 6,70% |

*Spectre de RMN*

pic à 1,6 ppm attribué aux hydrogènes des méthyles géminés,

pics à 2,0-3,17 ppm attribués aux hydrogènes en 3 et 4 du cyclopentyle,

pics à 5,25-5,28 ppm attribués à l'hydrogène en 5 du cyclopentyle,

pic à 3,5 ppm attribué aux hydrogènes du méthoxy.

*Stade B: Acide 3-formyl-4-méthylpent-3-én-1-oïque*

On dissout 18,9 g de carbonate de sodium dans 180 $cm^3$ d'eau, refroidit à 0°C, ajoute, en une seule fois, 18,2 g de la nitrolactone obtenue précédemment et dissoute dans 25 $cm^3$ de méthanol. Après 120 h à température ambiante, on lave le mélange réactionnel à l'éther éthylique, refroidit à 0°C, ajoute avec précaution et sous atmosphère inerte de l'acide sulfurique concentré jusqu'à pH≃1, extrait au chloroforme puis à l'acétate d'éthyle, sèche les phases organiques réunies, les concentre à sec sous pression réduite, pour obtenir 9,5 g de produit brut qui, par cristallisation dans l'eau, donnent 7,4 g de produit pur, F=102°C.

*Analyse* pour $C_7H_{10}O_3$=142,156

| | | |
|---|---|---|
| Calculé: | C 59,14 | H 7,09% |
| Trouvé: | C 59,20 | H 7,0 % |

*Spectre de RMN*

pic à 20 ppm attribué aux hydrogènes en 5,

pic à 2,26 ppm attribué aux hydrogènes du méthyle en 4,

pic à 3,38 ppm attribué aux hydrogènes en 2,

pic à 10,13 ppm attribué à l'hydrogène du formyle.

*Préparation 2: Acide 3-formyl-4-méthylpent-3-én-1-oïque*

*Stade A: 4-(2-Hydroxyprop-2-yl)-5-(3-phénoxyphényl)méthoxytétrahydrofuran-2-one-trans-dl*

On chauffe au reflux, sous agitation et sous atmosphère inerte, 3,5 g de 5-(3-phénoxyphényl)méthoxy-2,5-dihydrofuran-2-one dans 100 $cm^3$ d'isopropanol, ajoute régulièrement en 1½ h, par portions de 25 mg, 300 mg de peroxyde de benzoyle, concentre à sec sous pression réduite à 40/50°C, chromatographie le résidu sur silice puis élue avec un mélange benzène/acétate d'éthyle 8/2, recueille 3,7 g de produit brut qui, repris par l'éther isopropylique, donne des cristaux blancs, F=50/55°C.

*Analyse* pour $C_{20}H_{22}O_3$=342,39

| | | |
|---|---|---|
| Calculé: | C 70,16 | H 6,4% |
| Trouvé: | C 69,9 | H 6,5% |

*Spectre de RMN*

pics à 1,18 et 1,21 ppm attribués aux hydrogènes des méthyles,

pics à 2,16-2,75 ppm attribués aux hydrogènes en 3 et 4 du cyclopentyle,

pics à 4,48-4,35 et 4,8-5 ppm attribués aux hydrogènes du méthylène benzylique,

pics à 5,59-5,58 ppm attribués aux hydrogènes en 5 du cyclopentyle,

pics à 6,83-7,5 ppm attribués aux hydrogènes des noyaux aromatiques,

pic à 1,5 ppm attribué à l'hydrogène de l'hydroxyle.

La 5-(3-phénoxyphényl)méthoxy-2,5-dihydrofuran-2-one utilisée au départ de l'exemple a été préparée comme suit:

On mélange 12 g de 5-hydroxy-2-(5H)-furannone, 250 $cm^3$ de benzène, 25 g d'alcool métaphénoxybenzylique et 200 mg d'acide paratoluène sulfonique, agite en distillant du benzène en le remplaçant plusieurs fois par la même quantité de benzène sec, dans le mélange réactionnel, après 2 h, laisse refroidir à température ambiante et lave avec une solution saturée de bicarbonate de sodium puis à l'eau, sèche, concentre à sec sous pression réduite, obtient 39,7 g d'une huile que l'on purifie par chromatographie sur silice en éluant par un mélange benzène/acétate d'éthyle 9/1 et recueille 24,9 g du produit attendu.

*Spectre de RMN*

pics à 7,3-7,4 ppm attribués aux hydrogènes en 4 de la furanone,

pics à 6,18-6,32 ppm attribués aux hydrogènes en 3 de la furanone,

pic à 6,03 ppm attribué à l'hydrogène en 5 de la furanone,

pics à 4,58-4,76 et 4,85-5,12 ppm attribués aux hydrogènes du méthoxy,

pics à 6,92-7,5 ppm attribués aux noyaux aromatiques.

*Stade B: Acide 3-formyl-4-méthylpent-3-én-1-oïque*

On dissout 619 mg de carbonate de sodium dans 6 $cm^3$ d'eau, refroidit à 0°C, ajoute en agitant 1 g d'hydroxylactone obtenue précédemment et 2 $cm^3$ de méthanol, après 19 h de contact à température ambiante, lave à l'éther éthylique, refroidit à 0°C, ajoute lentement de l'acide

sulfurique concentré jusqu'à pH≃1, extrait au méthanol et au chloroforme, réunit les phases organiques, les sèche, les concentre à sec sous pression réduite, obtient 245 mg de produit brut qui, recristallisé, donne un produit fondant à 102°C.

*Préparation 3: Acide 3-formyl-4-méthylpent-3-én-1-oïque*

*Stade A: 4-(2-Paratoluènesulfonylprop-2-yl)-5-méthoxytétrahydrofuran-2-one-trans-dl*

On mélange 500 mg d'isopropylparatolylsulfone et 10 $cm^3$ de tétrahydrofuranne anhydre sous agitation et sous atmosphère inerte, refroidit à −70°C, ajoute lentement 1,3 $cm^3$ d'une solution cyclohexanique 1,95M de butyllithium, agite encore ½ h à −70°C, ajoute alors, en 10 min, 287 mg de 5-méthoxy-2,5-dihydrofuran-2-one en solution dans 4 $cm^3$ de tétrahydrofuranne, maintient pendant 1 h à −70°C, verse le mélange réactionnel sur une solution aqueuse de phosphate monosodique à 0°C, extrait au chlorure de méthylène, lave à l'eau, sèche, concentre à sec sous pression réduite, cristallise le résidu dans l'éther isopropylique et obtient 490 mg de cristaux blancs, F=129°C.

*Analyse* pour:

| | | | |
|---|---|---|---|
| Calculé: | C 57,67 | H 6,45 | S 10,26% |
| Trouvé: | C 57,6 | H 6,5 | S 10,1 % |

*Spectre de RMN*

pics à 1,27-1,32 ppm attribués aux hydrogènes des méthyles géminés,

pic à 2,46 ppm attribué aux hydrogènes du méthyle porté par l'aromatique,

pic à 2,75 ppm attribué aux hydrogènes 3 et 4 du cyclopentyle,

pic à 5,58 ppm attribué à l'hydrogène du cyclopentyle en 5,

pic à 3,51 ppm attribué aux hydrogènes du méthoxy,

pics à 7,28-7,42 ppm attribués aux hydrogènes en 3 et 5 de l'aromatique,

pics à 7,66-7,8 ppm attribués aux hydrogènes en 2 et 6 de l'aromatique.

*Stade B: Acide 3-formyl-4-méthylpent-3-én-1-oïque*

On agite 250 mg du produit obtenu précédemment, 4 $cm^3$ d'eau, 0,8 $cm^3$ de méthanol et 250 mg de carbonate de sodium pendant 2 d à température ambiante. Après lavage à l'éther, on acidifie la phase aqueuse à pH 3-3,5, avec de l'acide chlorhydrique N, sature de chlorure de sodium, extrait au chloroforme, sèche, concentre à sec sous pression réduite, et obtient 87 mg du produit attendu, F=102°C.

Ce produit est identique en tous points à l'acide 3-formyl-4-méthylpent-3-én-1-oïque obtenu par une autre voie.

*Préparation 4: Acide 3-formyl-4-méthylpent-3-én-1-oïque*

*Stade A: Iodure de [1-(5-méthoxy-2-oxo-4,5-dihydro-3H-furan-4-yl)-1-méthyl-1-éthyl]-triphénylphosphonium*

*a) Préparation de l'ylure*

A une suspension de 3 g d'iodure de triphénylisopropylphosphonium dans 40 $cm^3$ de tétrahydrofuranne, on ajoute en une fois 3,5 $cm^3$ de solution 2M de butyllithium dans le cyclohexane, agite pendant 10 min à température ambiante et obtient une solution d'ylure (solution A).

*b) Addition de l'ylure sur la furannone*

La solution A refroidie à −60°C est ajoutée lentement, sous atmosphère inerte, à une solution de 0,800 g de 5-méthoxy-2,5-dihydrofuran-2-one-3-ène dans 50 $cm^3$ de tétrahydrofuranne également refroidie à −60°C. On agite pendant 10 min, verse le mélange réactionnel sur un mélange de solution aqueuse de phosphate monosodique et de glace, lave à l'éther, extrait au chlorure de méthylène, sèche, concentre à sec sous pression réduite et obtient 3,4 g de sel de phosphonium, fondant vers 140°C.

*Analyse* pour $C_{26}H_8IO_3P$=546,37

| | | | |
|---|---|---|---|
| Calculé: | C 57,15 | H 5,16 | I 23,24 |
| | P 5,67% | | |
| Trouvé: | C 57,3 | H 5,2 | I 22,4 |
| | P 5,3 % | | |

*Spectre de RMN*

pics à 1,63-1,80-1,95-2,10 ppm attribués aux hydrogènes des méthyles en β du phosphore,

pics à 2,22-3,33 ppm attribués aux hydrogènes portés par les carbones en position 3 et 4 de la furannone,

pic à 3,23 ppm attribué aux hydrogènes du méthoxy,

pics à 5,46-5,54 ppm attribués à l'hydrogène en 5 de la furannone,

pics à 7,81-7,92 ppm attribués aux hydrogènes des noyaux aromatiques.

*Stade B: Acide 3-formyl-4-méthylpent-3-én-1-oïque*

A une solution de 0,700 g d'iodure de [1-(5-méthoxy-2-oxo-4,5-dihydro-3H-furan-4-yl)-1-méthyl-1-éthyl]triphénylphosphonium dans 1 $cm^3$ de méthanol, on ajoute 0,700 g de carbonate de sodium en solution dans 8 $cm^3$ d'eau, agite pendant 2 h à 20°C, élimine par filtration l'insoluble formé, acidifie le filtrat à pH 2, sature par du chlorure de sodium, extrait au chloroforme, concentre à sec, purifie dans l'éther isopropylique et obtient 0,095 g d'acide 3-formyl-4-méthylpent-3-én-1-oïque, F=102°C.

*Application 1: dl-6,6-Diméthyl-4-hydroxy-3-oxabicyclo-[3.1.0]-hexan-2-one*

On refroidit, vers −20°C, 0,55 $cm^3$ d'une solution molaire de diisopropylamine dans du tétrahydrofuranne et 5 $cm^3$ de tétrahydrofuranne, ajoute 0,25 $cm^3$ d'une solution de n-butyllithium 2M dans du cyclohexane, laisse remonter la température à 0°C, puis refroidit à −60 à −70°C,

ajoute en une seule fois 180 mg du produit obtenu à l'exemple 3, après 2 h de réchauffement jusqu'à 0°C, maintient 1 h cette température, verse le mélange réactionnel dans de l'acide chlorhydrique 2N glacé et laisse 17 h à 20°C sous vive agitation, décante, extrait au chloroforme, sèche, concentre à sec, reprend le résidu obtenu par un mélange éther isopropylique/éther de pétrole, extrait à l'eau, concentre à sec sous pression réduite la phase aqueuse pour obtenir 30 mg de produit cristallisé, F=80°C.

*Application 2: 6,6-Diméthyl-4-hydroxy-3-oxabicyclo-[3.1.0]-hexan-2-one*

On refroidit, vers −20°C, 0,55 cm³ d'une solution molaire de diisopropylamine dans du tétrahydrofuranne diluée avec 5 cm³ de tétrahydrofuranne, ajoute 0,25 cm³ d'une solution de n-butyllithium 2M dans le cyclohexane, laisse réchauffer le mélange vers 0°C, puis refroidit à −60°C pour ajouter en une seule fois 200 mg du dérivé bromé obtenu à l'exemple 4, après 15 min de réaction à cette température on obtient une solution de 6,6-diméthyl-4-[(3-phénoxyphényl)-méthoxy]-3-oxabicyclo-[3.1.0]-hexan-2-one-dl, la verse sur de l'acide chlorhydrique 2N glacé et laisse 17 h à 20°C sous vive agitation, décante, extrait au chloroforme, sèche, concentre à sec, obtient 170 mg de produit, le reprend par un mélange éther isopropylique/éther de pétrole, l'extrait à l'eau, concentre la phase aqueuse à sec sous pression réduite pour obtenir 50 mg de produit qui cristallise, F=80°C.

*Application 3: (1R,5S)-6,6-diméthyl-4-hydroxy-3-oxabicyclo-[3.1.0]-hexan-2-one*

*Stade A: (1R,5S)-6,6-diméthyl-4(R)[(S)(3-phénoxyphényl)méthylméthoxy]-3-oxabicyclo-[3.1.0]-hexan-2-one*

Pendant 2½ h à température ambiante, on agite 300 mg de dérivé bromé cristallisé (isomère B) obtenu à l'exemple 5, 3 cm³ de chlorure de méthylène, 3 cm³ d'une solution aqueuse d'hydroxyde de sodium à 50% (P/p) et environ 30 mg de chlorure de triéthylbenzylammonium, verse le mélange réactionnel sur une solution aqueuse glacée de phosphate monosodique, l'extrait au chlorure de méthylène, la lave à l'eau, la sèche, la concentre à sec sous pression réduite, obtient ≃221 mg de produit brut qui cristallise, recristallise le produit dans 4 cm³ d'un mélange éther de pétrole/éther isopropylique 7/3, obtient 165 mg de produit blanc, F≃110°C. Les liqueurs mères chromatographiées donnent 17 mg de cristaux blancs, F≃110°C.
$(\alpha)_D = -241°$ (1% benzène).

*Spectre de RMN*

pics à 1,1 et 1,13 ppm attribués aux hydrogènes des méthyles géminés,

pic à 2,03 ppm attribué aux hydrogènes en 1 et 5,

pic à 4,96 ppm attribué à l'hydrogène en position 4,

pics à 1,42 et 1,53 ppm attribués aux hydrogènes du méthyle fixé sur le carbone benzylique,

pics à 4,7-4,8-4,9 et 5,0 ppm attribués à l'hydrogène porté par le carbone benzylique,

pics à 6,83-7,5 ppm attribués aux hydrogènes des noyaux aromatiques.

*Stade B: (1R,5S)-6,6-diméthyl-4-hydroxy-3-oxabicyclo-[3.1.0]-hexan-2-one*

Pendant 2 h, à 20/25°C, on agite 1 g du produit obtenu précédemment, 10 cm³ d'acétone et 5 cm³ de solution aqueuse normale d'acide chlorhydrique, amène le mélange réactionnel à pH 8 avec du bicarbonate de sodium, le lave au chlorure de méthylène, acidifie la phase aqueuse à pH 1 avec de l'acide chlorhydrique concentré, extrait à l'acétate d'éthyle, sèche et évapore le solvant sous pression réduite. On obtient 384 mg de cristaux, F=117°C.

Produit identique au produit obtenu dans le brevet français N° 1580474 $(\alpha)_D = -110°$ (c=1% diméthylformamide).

*Application 4: 6,6-Diméthyl-4-hydroxy-3-oxabicyclo-[3.1.0]-hexane-2-one-dl*

*Stade A: 6,6-Diméthyl-4-isopropyl-3-oxabicyclo-[3.1.0]-hexan-2-one-dl*

On mélange 728 mg du produit obtenu à l'exemple 6, 5 cm³ de tétrahydrofuranne anhydre et 1 cm³ de diméthylsulfoxyde anhydre, refroidit à 0°C, ajoute en une seule fois 150 mg d'hydrure de sodium, après 20 h d'agitation à température ambiante et sous atmosphère inerte, verse sur une solution aqueuse de phosphate monosodique, extrait au benzène, sèche, concentre à sec sous pression réduite et obtient 450 mg de produit brut, le chromatographie sur silice et élue par le système de solvant éther de pétrole/éther éthylique 7/3, obtient 350 mg du produit attendu.

*Spectre de RMN*

pics à 1,17-1,31 ppm attribués aux hydrogènes des méthyles en 6 et des méthyles de l'isopropyle,

pic à 2,02 ppm attribué aux hydrogènes en 1 et 5 du cycle,

pic à 5,25 ppm attribué à l'hydrogène en 4 du cycle,

pic à 4,03 ppm attribué à l'hydrogène en 2 du propyle.

*Stade B: 6,6-Diméthyl-4-hydroxy-3-oxabicyclo-[3.1.0]-hexan-2-one-dl*

On agite 150 mg du produit obtenu précédemment avec 3 cm³ d'acétone et 1 cm³ d'acide chlorhydrique aqueux N pendant 4 h à 20/25°C, sature le mélange réactionnel avec du chlorure de sodium, extrait au chlorure de méthylène, sèche la phase organique, évapore à sec sous pression réduite et obtient 95 mg de cristaux blancs fondant à 80°C.

**Revendications:**

1. Les composés de formule générale (VIII):

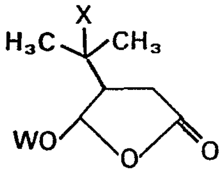

(VIII)

dans laquelle X représente un atome d'halogène et notamment un atome de brome ou de chlore et W représente de l'hydrogène ou un radical $R_1$, $R_1$ représentant le reste d'un alcool $R_1OH$, éventuellement chiral.

2. Procédé de préparation des composés de formule générale (VIII) tels que définis à la revendication 1, caractérisé en ce que l'on fait réagir l'acide 3-formyl-4-méthylpent-3-én-1-oïque, en milieu anhydre avec un hydracide HX, dans lequel X représente un atome d'halogène, en présence d'un hydrogénure du lithium LiX, au sein d'un solvant organique pour obtenir un composé de formule (VIII'):

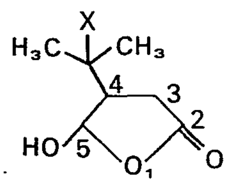

(VIII')

que l'on fait réagir avec un alcool $R_1OH$ achiral pour obtenir le composé (VIII'') racémique:

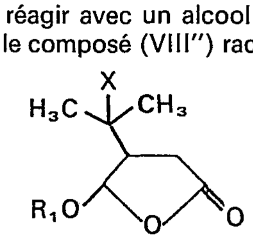

(VIII'')

qui, comme le dérivé (VIII'), présente une relation trans entre les substituants en 4 et en 5 de la tétrahydrofurannone, soit avec un alcool $R_1OH$ chiral, le composé (VIII'') étant alors obtenu sous la forme d'un mélange des deux diastéréo-isomères prévisibles, mélange que l'on peut séparer en ses constituants par des procédés physiques.

3. Application des composés de formule générale (VIII') tels que définis à la revendication 2 à la préparation des composés de formule (V'A):

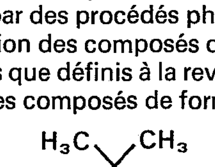

(V'A)

caractérisé en ce que l'on soumet un composé de formule (VIII') à l'action d'un alcool $R_1OH$ pour obtenir un composé de formule (VIII'') que l'on soumet à l'action d'un agent basique pour obtenir un composé bicyclique de formule (V'):

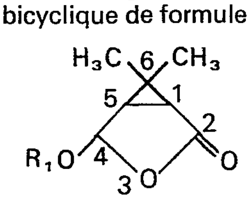

(V')

sous forme racémique ou sous l'une ou l'autre de ses formes énantiomériques selon que $R_1$ est respectivement un reste achiral ou chiral, la configuration absolue en position 4 de ce composé ainsi que celles qui en découlent en position 1 et 5 étant alors déterminées par la stéréochimie du diastéréoisomère (VIII'') mis en œuvre, puis hydrolyse l'éther de formule (V') obtenu en milieu acide, avec rétention totale des configurations absolues, pour obtenir le composé de formule (V'A) correspondant:

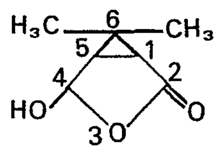

(V'A)

**Patentansprüche**

1. Die Verbindungen der allgemeinen Formel (VIII)

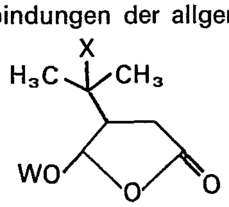

(VIII)

worin X ein Halogenatom, und insbesondere ein Brom- oder Chloratom bedeutet und W Wasserstoff oder einen Rest $R_1$ darstellt, wobei $R_1$ den Rest eines gegebenenfalls chiralen Alkohols $R_1OH$ darstellt.

2. Verfahren zur Herstellung der Verbindungen der allgemeinen Formel VIII gemäss Anspruch 1, dadurch gekennzeichnet, dass man 3-Formyl-4-methylpent-3-en-1-säure in wasserfreiem Milieu mit einer Wasserstoffsäure HX, worin X ein Halogenatom bedeutet, in Gegenwart eines Hydrids von Lithium LiX in dem Medium eines organischen Lösungsmittels, umsetzt, um eine Verbindung der Formel (VIII')

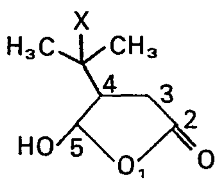

(VIII')

zu erhalten, die man mit einem achiralen Alkohol $R_1OH$ umsetzt, um die racemische Verbindung (VIII'')

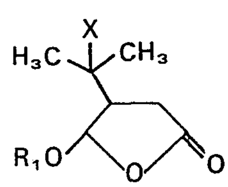

(VIII'')

zu erhalten, die wie das Derivat (VIII') eine trans-Beziehung zwischen den Substituenten in 4- und 5-Stellung des Tetrahydrofuranons besitzt oder mit einem chiralen Alkohol $R_1OH$ umsetzt, wobei die Verbindung (VIII'') dann in Form eines Gemisches der beiden zu erwartenden Diastereomeren erhalten wird, welches Gemisch man nach physikalischen Verfahren in seine Bestandteile auftrennen kann.

3. Verwendung der Verbindungen der allgemeinen Formel (VIII') gemäss Anspruch 2 zur Herstellung von Verbindungen der Formel (V'A)

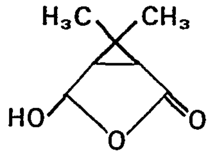

(V'A)

dadurch gekennzeichnet, dass man eine Verbindung der Formel (VIII') der Einwirkung eines Alkohols $R_1OH$ unterzieht, um eine Verbindung der Formel (VIII") zu erhalten, die man der Einwirkung eines basischen Mittels unterzieht, um eine bicyclische Verbindung der Formel (V')

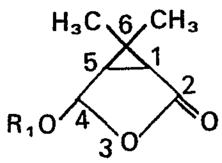

(V')

in racemischer Form oder in der einen oder anderen ihrer enantiomeren Formen, je nachdem, ob $R_1$ ein achiraler oder chiraler Rest ist, zu erhalten, wobei die absolute Konfiguration in 4-Stellung dieser Verbindung sowie diejenigen, die von der 1- und 5-Stellung herrühren, dann von der Stereochemie des eingesetzten Diastereomeren (VIII") bestimmt werden, danachden erhaltenen Äther der Formel (V') in saurem Milieu unter vollständiger Beibehaltung der absoluten Konfigurationen hydrolysiert, um die entsprechende Verbindung der Formel (V'A)

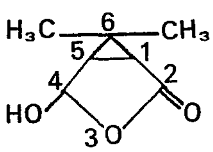

(V'A)

zu erhalten.

**Claims:**

1. The compounds with the general formula (VIII):

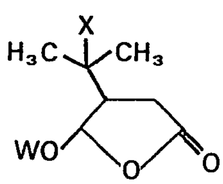

(VIII)

in which X represents a halogen atom and notably a bromine or chlorine atom and W represents hydrogen or an $R_1$ radical, $R_1$ representing the residue of an $R_1OH$ radical, possibly chiral.

2. Preparation process for the compounds with the general formula (VIII) as defined in Claim 1, characterized in that 3-formyl-4-methylpent-3-en-1-oic acid is made to react, in an anhydrous medium, with an HX hydracid, in which X represents a halogen atom, in the presence of a lithium hydride LiX, in an organic solvent, so as to obtain a compound with the formula (VIII'):

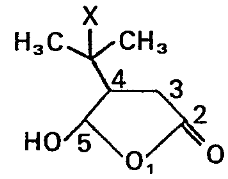

(VIII')

which is made to react with an $R_1OH$ achiral alcohol so as to obtain the racemic compound (VIII"):

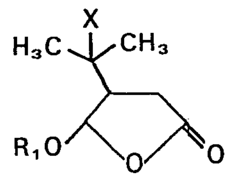

(VIII")

which, like the derivative (VIII'), displays a trans relationship between the substituents in position 4 and in position 5 of the tetrahydrofuranone, or with an $R_1OH$ chiral alcohol, the compound (VIII") then being obtained in the form of a mixture of the two foreseeable diastereoisomers, which mixture may be separated into its constituents by physical processes.

3. Use of the compounds with the general formula (VIII') as defined in Claim 2 in the preparation of compounds with the formula (V'A):

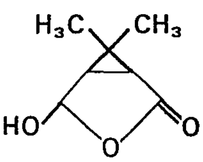

(V'A)

characterized in that a compound with the formula (VIII') is submitted to the action of an $R_1OH$ alcohol so as to obtain a compound with the formula (VIII") which is submitted to the action of a base agent so as to obtain a bicyclic compound with the formula (V'):

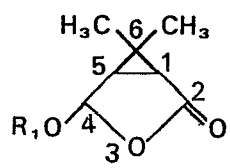

(V')

in racemic form or in one or other of its enantiomeric forms according to whether $R_1$ is an achiral or chiral residue respectively, the absolute configuration in position 4 of this compound as well as those resulting from it in position 1 and 5 thus being determined by the stereochemistry of the diastereoisomer (VIII") being used, then the ether of the formula (V') obtained is hydrolized in acid medium, with total retention of the absolute configurations, so as to obtain the corresponding compound with the formula (V'A):

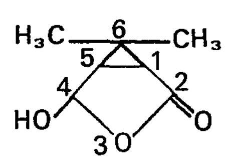

(V'A)